**Europäisches Patentamt**

**European Patent Office**

**Office européen des brevets**

(19)

(11) Veröffentlichungsnummer: **0 120 213**
**B1**

(12)

# EUROPÄISCHE PATENTSCHRIFT

(45) Veröffentlichungstag der Patentschrift:
28.01.87

(51) Int. Cl.⁴: **C 12 P 7/46** // (C12P7/46,
C12R1:66, 1:79, 1:80, 1:645)

(21) Anmeldenummer: **84100768.5**

(22) Anmeldetag: **21.01.84**

(54) Verfahren zur biotechnologischen Herstellung von L-Äpfelsäure.

(30) Priorität: **25.03.83 DE 3310849**

(43) Veröffentlichungstag der Anmeldung:
**03.10.84 Patentblatt 84/40**

(45) Bekanntmachung des Hinweises auf die Patenterteilung:
**28.01.87 Patentblatt 87/5**

(84) Benannte Vertragsstaaten:
**AT BE CH DE FR GB IT LI LU NL SE**

(56) Entgegenhaltungen:
**DE - A - 1 417 033**
**FR - A - 2 223 351**
**US - A - 3 063 910**
**US - A - 3 922 195**

**CHEMICAL ABSTRACTS, Band 87, Nr. 23, 5. Dezember
1977, Seite 464, Nr. 182622f, Columbus, Ohio, US**

(73) Patentinhaber: **HÜLS AKTIENGESELLSCHAFT,
Patentabteilung / PB 15 - Postfach 13 20,
D-4370 Marl 1 (DE)**

(72) Erfinder: **Schindler, Fritz, Dr., Am Stadtgarten 1/1661,
D-4650 Gelsenkirchen (DE)**

## Beschreibung

Die Erfindung betrifft ein Verfahren zur Herstellung des reinen L-Isomeren der Äpfelsäure aus neutralisierter Fumarsäure durch mikrobielle Fermentation mittels frei beweglicher Mikroorganismen in wässriger Phase und einer Konzentration der L-Äpfelsäure zum Zeitpunkt der Ernte von 100 bis 170 g pro Liter der Kulturlösung.

L-Äpfelsäure wird in der Lebensmittel- und pharmazeutischen Industrie als Puffersubstanz, Komplexbildner, Acidulans und Feuchthaltemittel eingesetzt. L-Äpfelsäure ist als Naturstoff für diese Anwendungsgebiete geeigneter als das chemisch, z. B. durch Wasseranlagerung an Maleinsäureanhydrid, zugängliche D,L-Isomerengemisch der Äpfelsäure. Das D-Isomer der Äpfelsäure kommt in der Natur nicht vor, weshalb die Anwendung der chemisch synthetisierten D,L-Äpfelsäure im Lebensmittel- und Pharmabereich nicht unbedenklich ist.

Zweck der vorliegenden Erfindung ist die Verbesserung der Wirtschaftlichkeit der biotechnologischen Herstellung von L-Äpfelsäure in reiner Form.

Zur biotechnologischen Herstellung von L-Äpfelsäure sind mehrere Verfahren bekannt. Ein Teil dieser Verfahren arbeitet mit speziellen Bakterien, die Fumarsäure durch Anlagerung von Wasser in L-Äpfelsäure überführen. In US-3.922.195 wird vorgeschlagen, die Bakterienzellen zu immobilisieren. Nach DE-AS 2363285 wird mit freien Bakterienzellen oder mit dem aus Bakterienzellen isolierten Enzym Fumarase gearbeitet.

Weiter ist die Umsetzung von Glucose in L-Äpfelsäure durch Vergesellschaftung eines Pilzes mit einem Bakterium beschrieben worden (J. Ferment. Technol., Vol. 54, No. 4 (1976), Seiten 197 bis 204). Hierbei bildet der Pilz aus Glucose Fumarsäure, die dann von dem Bakterium in L-Äpfelsäure überführt wird.

Das die Umsetzung von Fumarsäure in L-Äpfelsäure bewirkende Enzym, die Fumarase, kann aus biologischem Material, wie der Zellmasse von Bakterien oder Pilzen, in speziellen Verfahren isoliert und dann – in freier oder immobilisierter Form – zur Gewinnung von L-Äpfelsäure aus Fumarsäure eingesetzt werden (DE-AS 2415310; CS-171.990).

Bei einigen Verfahren wird dem Fermentationsansatz eine grosse Menge einer Kalziumverbindung zugegeben, wodurch die gebildete L-Äpfelsäure bereits während der Fermentation als Ca-Malat ausfällt (DE-OS 1417033).

Verfahren zur biotechnologischen Herstellung von L-Äpfelsäure mit Hilfe von Pilzen sind ebenfalls bekannt. Bei diesen Verfahren wird die L-Äpfelsäure im wesentlichen durch biochemischen Ab- bzw. Umbau der an den betreffenden Pilz verfütterten Kohlenstoffquelle (z. B. Melasse, Zukker, Ethanol, Essigsäure) erhalten (J. Ferment. Technol. Vol. 55, No. 2 (1977), Seiten 196 bis 199). Diese Verfahren gehen nicht direkt von Fumarsäure aus.

Bei dem in US-3.063.910 beschriebenen Verfahren kann der Fermentationsansatz neben 10 bis 15% eines Zuckers noch 1 bis 10% einer organischen Säure, wie z. B. Brenztraubensäure oder Fumarsäure, enthalten. Jedoch dient bei diesem Pilzverfahren die zugesetzte organische Säure nicht als Substrat für die Synthese von L-Äpfelsäure, sondern als Reaktionsbeschleuniger des biochemischen Ab- bzw. Umbaues des angebotenen Zuckers in L-Äpfelsäure.

Weiter ist die fermentative Überführung von n-Paraffin in L-Äpfelsäure durch Vergesellschaftung von zwei Hefearten möglich, wobei die eine Hefeart Paraffin in Fumarsäure überführt und die zweite Hefeart die entstandene Fumarsäure in L-Äpfelsäure umwandelt (Agr. Biol. Chem., Vol. 34 (1970), Seiten 1833 bis 1838).

Die bekannten Verfahren zur biotechnologischen Herstellung von L-Äpfelsäure haben u.a. die nachstehend aufgeführten Eigenarten:

– Bei der Verwendung von Bakterien sind biotechnologische Verfahren schwieriger zu handhaben als bei Verwendung von Pilzen. Aus der geringen Grösse der Bakterien können beispielsweise Abtrennprobleme entstehen. Bakterien neigen ferner dazu, Nebenprodukte, darunter auch toxische Nebenprodukte, zu bilden. Besonders störend für eine Anwendung der L-Äpfelsäure im Pharmabereich ist das von Bakterien gebildete LPS-Toxin (Lipopolysaccharid-Toxin), das pyrogen wirkt, und von dem die L-Äpfelsäure nur durch aufwendige Verfahrensschritte, wie z. B. durch Ultrafiltration, zu reinigen ist. Bei dem in US-3.922.195 beschriebenen Bakterienverfahren tritt als Nebenprodukt in geringer Menge Bernsteinsäure auf, die von Äpfelsäure nur mit Schwierigkeiten abzutrennen ist.

– Beim Einsatz des Enzyms Fumarase kommt als Fumarasequelle in erster Linie mikrobielle Zellmasse in Frage. Vor Beginn der Herstellung von L-Äpfelsäure muss mikrobielle Zellmasse gezüchtet und mit erheblichem Aufwand zu Fumarase aufgearbeitet werden.

– Die Immobilisierung von lebenden Zellen oder des aus Zellen isolierten Enzyms Fumarase ist ein zusätzlicher, sehr aufwendiger Verfahrenschritt.

– Bei der Fermentation in Gegenwart von CaIonen in grosser Konzentration wird die gebildete L-Äpfelsäure bereits während der Fermentation als Ca-Malat gefällt; das führt zu Problemen beim Rühren und Belüften des Fermentationsansatzes.

– Die erzielbare Konzentration der L-Äpfelsäure ist im allgemeinen vergleichsweise gering und liegt deutlich unter 100 g pro Liter Kulturlösung, wenn man von den in US-3.922.195 (immobilisierte Bakterien) und in DE-OS 1417033 (hohe Kalziumkonzentration) beschriebenen Umsetzungen absieht. Die Fermentation dauert länger als drei Tage. Die wegen dieser geringen Produktivität erforderliche grosse Kapazität der Bio-Reaktoren verursacht einen grossen apparativen Aufwand, zumal es sich um aufwendig gebaute Reaktoren handelt, die eine kontaminationsfreie Prozessführung ermöglichen.

Damit stellt sich die Aufgabe, ein Verfahren der obengenannten Art zu entwickeln, bei dem L-Äpfelsäure mit grosser Produktivität in Bio-Reaktoren einfacher Bauart in einer der Aufarbeitung zu reiner L-Äpfelsäure entgegenkommenden Form auf möglichst wirtschaftliche Weise hergestellt wird.

Diese Aufgabe wird erfindungsgemäss gelöst durch ein Verfahren, das durch folgende Merkmale gekennzeichnet ist:

– Verwendung von Pilzen als Mikroorganismen,

– eine Konzentration der vorgelegten Fumarsäure von 11 bis 15 Gew.-%.

Die Fumarsäure kann in nährstoffhaltiger oder nährstofffreier Lösung zu L-Äpfelsäure fermentiert werden. Es kann zweckmässig sein, die Fumarsäure nur in nährstofffreier Lösung zu fermentieren und die dazu benötigte Zellmasse in einer fumarsäurefreien oder fumarsäurehaltigen Nährlösung zu züchten. Die dazu geeignete Nährlösung kann wesentlich weniger als 11% Fumarsäure enthalten, da in diesem Fall der Pilz lediglich an Fumarsäure adaptiert werden soll.

Obwohl der Begriff «Fermentation» in der Fachliteratur bisher einen relativ grossen Bedeutungsumfang hat, wird in diesem Zusammenhang unter «Fermentation» die biochemische Umwandlung von Fumarsäure in L-Äpfelsäure unter Einwirkung der Fumarase verstanden. Mit «Züchtung» wird die Vermehrung der Biomasse aus dem Inoculum unter Verbrauch von Nährstoffen bezeichnet. In nährstoffhaltiger Fumarsäurelösung laufen beide Vorgänge (Züchtung und Fermentation) neben- bzw. hintereinander ab.

Für die Fermentation in nährstoffhaltiger Fumarsäurelösung werden der Lösung mit neutralisierter Fumarsäure die für das Wachsen des Pilzes nötigen Nähr- und Wirkstoffe zugesetzt. Nach ihrer Sterilisation wird diese Lösung mit 1 bis 20% Inoculum eines Pilzes geimpft und während 1 bis 3 Tagen bei 20 bis 50 °C fermentiert. Anschliessend wird die Kulturlösung in Zellmasse und Kulturfiltrat getrennt, und aus dem Kulturfiltrat wird nach den bekannten Verfahren die L-Äpfelsäure isoliert.

Die abgetrennte Zellmasse kann für mehrere – etwa für drei weitere – Fermentationen in jeweils neu angesetzter nährstofffreier Fumarsäurelösung verwendet werden. Dabei kann das Volumen der Fumarsäurelösung das Einfache bis Zehnfache des Volumens der vorhergehenden Fermentation in nährstoffhaltiger Lösung oder des Volumens der Züchtung in fumarsäurefreier oder fumarsäurehaltiger Nährlösung ausmachen, wenn die gesamte aus der nährstoffhaltigen Lösung resultierende Zellmasse verwendet wird. In nährstofffreier Fumarsäurelösung wird 12 bis 24 Stunden lang bei 25 bis 60 °C fermentiert.

Die aerobe fermentative Umsetzung – in nährstoffhaltiger Lösung – von Fumarsäure in L-Äpfelsäure erfolgt unter Belüften in einer einfach zusammengesetzten anorganischen Nährsalzlösung, die als Kohlenstoffquelle für den Energie- und Betriebsstoffwechsel des Pilzes Maismehl, einen Zucker oder eine andere Kohlenstoffverbindung (z. B. Ethanol) und als Substrat für die L-Äpfelsäureproduktion 11 bis 15 Gew.-% Fumarsäure in Form eines Salzes enthält.

Bei dem verwendeten Pilz handelt es sich vorzugsweise um einen Pilz, der zwar Fumarsäure in L-Äpfelsäure überführen kann, der aber weder Fumarsäure noch L-Äpfelsäure in einem nennenswerten Ausmass für seinen Energie- und Betriebsstoffwechsel nutzt. Dazu gehören Pilze, die bevorzugt aus den Ordnungen Aspergillus, Penicillium, Paecilomyces, Trichosporon, Taphrina, Helminthosporium und Pythium stammen. Das erfindungsgemässe Verfahren ist jedoch nicht auf Pilze aus diesen Ordnungen beschränkt.

Wegen der grossen Produktivität kann die Fermentation in nährstoffhaltiger Fumarsäurelösung nach spätestens drei Tagen abgebrochen werden. Die Fermentation in nährstofffreier Fumarsäurelösung kann bereits nach 12 bis 24 Stunden abgebrochen werden. Die Konzentration der L-Äpfelsäure in der Suspension beträgt zum Zeitpunkt der Ernte in jedem Fall mehr als 100 g pro Liter.

Die L-Äpfelsäure wird weitgehend – oder ausschliesslich – in nährstofffreier Fumarsäurelösung hergestellt. Pilz-Zellmasse aus einer vorhergehenden Fermentation oder Züchtung wird hierzu nach ihrer erfolgten (Fermentation) bzw. erfolgten oder nicht erfolgten (Züchtung) Abtrennung vom Kulturfiltrat in eine Lösung, die lediglich Fumarsäure (11 bis 15 Gew.-%) in Form eines Salzes enthält, überführt und unter leichtem Rühren und Belüften erneut inkubiert. Nach 12 bis 24 Stunden liefert diese Fermentation L-Äpfelsäure in einer Konzentration von mehr als 100 g pro Liter. Mit der aus einer Fermentation oder Züchtung in nährstoffhaltiger Lösung resultierenden Pilz-Zellmasse können mehrere – etwa bis zu drei – Fermentationen in nährstofffreier Fumarsäurelösung nacheinander durchgeführt werden, wobei das Flüssigkeitsvolumen jeder einzelnen dieser Fermentationen das Einfache bis Zehnfache – vorzugsweise das Einfache bis Dreifache – des Volumens der vorhergehenden Fermentation in nährstoffhaltiger Fumarsäurelösung (oder der Züchtung in fumarsäurefreier oder fumarsäurehaltiger Nährstofflösung) beträgt.

Die Bio-Reaktoren für die Fermentation in nährstofffreier Fumarsäurelösung sind wesentlich einfacher gebaut als übliche Bio-Reaktoren. Sie benötigen beispielsweise – da in ihnen der Pilz nicht mehr wächst – kein leistungsfähiges Rühr- und Belüftungssystem zur Erzielung einer grossen Sauerstoff-Transferrate. Eine Vorrichtung zur Sterilisation ist ebenfalls nicht erforderlich, da diese Fermentationen mit 12 bis 24 Stunden Dauer vergleichsweise kurz sind und Infektionskeime – die wegen des Fehlens einer leicht metabolisierbaren Kohlenstoffverbindung und aufgrund des grossen osmotischen Druckes der Lösung ohnehin ungünstige Wachstumsbedingungen haben – sich in dieser kurzen Zeit nicht nennenswert entwickeln können.

Die nährstofffreie Lösung enthält als einzige Komponente Fumarsäure in Form eines Salzes und gegebenenfalls Aktivatoren für die fermentative Umsetzung von Fumarsäure in L-Äpfelsäure. In dieser Lösung ist der Stoffwechsel des Pilzes reduziert auf die Umwandlung von Fumarsäure in L-Äpfelsäure, die somit frei von den üblicherweise in einer Kulturlösung enthaltenen Komponenten – wie z. B. den für das Wachstum zugesetzten Nährstoffen und den als Nebenprodukte der Wachstumsvorgänge entstehenden organischen Verbindungen – anfällt.

Die Fumarsäure ist ein in Nahrungsmittelqualität grosstechnisch hergestelltes Massenprodukt, das für das erfindungsgemässe Verfahren unmittelbar eingesetzt werden kann.

Bei der Fermentation in nährstoffhaltiger Fumarsäurelösung geht man folgendermassen vor.

Fumarsäure wird in einer Konzentration von 11 bis 15%, vorzugsweise von 12 bis 13%, in Wasser vorgelegt und mit Lauge, vorzugsweise Natronlauge neutralisiert. Die Lösung wird mit den für das Wachsen des Pilzes notwendigen anorganischen Nährstoffen versetzt, beispielsweise pro Liter Lösung mit

1 ml konzentrierter Phosphorsäure ($H_3PO_4$)
1,4 ml konzentrierter Ammoniumhydroxidlösung ($NH_4OH$)
0,9 g Magnesiumsulfat ($MgSO_4 \cdot 7\,H_2O$)
30 mg Eisenchlorid ($FeCl_3 \cdot 6\,H_2O$)
2 g Kaliumchlorid (KCl)

Der Zusatz von KCl ist erforderlich, wenn nicht zumindest ein Teil der Fumarsäure mittels Kaliumhydroxid neutralisiert wurde. Für Ansätze mit Pilzen, die zum Wachsen bestimmte Wirkstoffe (z. B. Vitamine) benötigen, wird die Lösung noch mit diesen Wirkstoffen versetzt.

Die nährstoffhaltige Lösung wird auf einen pH-Wert von 3 bis 9, vorzugsweise von 6 bis 8, eingestellt und anschliessend – vorzugsweise thermisch – sterilisiert. Die zum Wachsen des Pilzes erforderlichen Kohlenstoffverbindungen werden entweder zusammen mit den anorganischen Nährstoffen sterilisiert oder – z. B. zur Vermeidung von Karamelisierungserscheinungen bei Zuckern – separat davon. Geeignete Kohlenstoffverbindungen für das Pilzwachstum sind Maismehl, Saccharose, Glucose, Glycerin, Ethanol und andere kohlenstoffhaltige Verbindungen.

Nach der Sterilisation wird der Fermenter mit Inoculum eines für die Umsetzung von Fumarsäure in L-Äpfelsäure geeigneten Pilzes in einer Menge von 1 bis 20%, vorzugsweise in einer Menge von 3 bis 10%, des Fermentationsansatzes angeimpft und unter kontrollierten Bedingungen hinsichtlich Temperatur, Belüftung und pH-Wert für 1 bis 3 Tage betrieben. Während dieser Zeit baut der eingeimpfte Pilz zunächst unter Verbrauch der zugesetzten Kohlenstoffverbindung seine Zellmasse auf, die dann den weitaus grössten Teil der vorgelegten Fumarsäure in L-Äpfelsäure überführt, so dass nach spätestens drei Tagen eine L-Äpfelsäure-Konzentration von mehr als 100 g pro Liter Kulturlösung erhalten wird. Die Fermentationstemperatur liegt bei 20 bis 50 °C, vorzugsweise bei 25 bis 35 °C. Sie kann, wenn das Wachsen des Pilzes weitgehend beendet ist (beispielsweise nach 1,5 Tagen Fermentation), auf 25 bis 60 °C, vorzugsweise auf 30 bis 45 °C, erhöht werden. Aus dieser Temperaturerhöhung nach Beendigung des Pilzwachstums resultiert eine Erhöhung der Umsatzgeschwindigkeit von Fumarsäure in L-Äpfelsäure, denn überraschenderweise liegt die optimale Temperatur für die Umsetzung von Fumarsäure in L-Äpfelsäure über der für das Wachsen des Pilzes optimalen Temperatur.

Der pH-Wert während der Fermentation wird durch Einspeisen des entsprechenden Neutralisationsmittels zwischen 3 und 9, vorzugsweise zwischen 6 und 8, gehalten. Rührerdrehzahl und Belüftung sind auf ausreichende Versorgung des Pilzes mit Sauerstoff einzustellen.

Nach Beendigung der Fermentation wird die Kulturlösung durch Filtrieren oder eine andere Flüssigkeit/Feststoff-Trennoperation in Kulturfiltrat und Pilz-Zellmasse getrennt. Das Filtrat steht zur Isolierung der in ihm enthaltenen L-Äpfelsäure nach bekannten Verfahren, beispielsweise durch Fällung als Ca-Malat, zur Verfügung. Die Pilz-Zellmasse kann zur anschliessenden Fermentation in nährstofffreier Fumarsäurelösung eingesetzt werden.

Bei der Fermentation in nährstofffreier Fumarsäurelösung geht man folgendermassen vor.

Die aus einer vorhergehenden Fermentation in nährstofffreier oder nährstoffhaltiger Fumarsäurelösung oder aus einer Züchtung in fumarsäurefreier oder fumarsäurehaltiger Nährstofflösung resultierende Zellmasse wird in eine Lösung, die lediglich 11 bis 15% (vorzugsweise 12 bis 13%) Fumarsäure in Form eines Salzes enthält, überführt; die Suspension wird in einem Bio-Reaktor unter schwachem Rühren und Belüften für 12 bis 24 Stunden inkubiert. Die Konzentration an L-Äpfelsäure liegt dann – wie am Ende der Fermentation in nährstoffhaltiger Fumarsäurelösung – bei mehr als 100 g pro Liter Suspension. Die Temperatur während der Fermentation in nährstofffreier Fumarsäurelösung liegt bei 25 bis 60 °C, vorzugsweise bei 30 bis 45 °C.

Die Fermentation in nährstofffreier Fumarsäurelösung kann in einem Bio-Reaktor von sehr einfacher Bauart durchgeführt werden; dieser Reaktor benötigt keine Vorrichtungen zur Erzielung einer hohen Sauerstoff-Transferrate und der Sterilisation. Es genügt ein einfach zu reinigender thermostatisierbarer Behälter mit Vorrichtungen zum Rühren und Belüften. Ein schwaches Rühren ist erforderlich, um die Sedimentation der Pilz-Zellmasse zu verhindern; ein schwaches Belüften ist notwendig, um die Zellen mit der für die Erhaltungsatmung nötigen Sauerstoffmenge zu versorgen.

Die einfache Bauart des Bio-Reaktors für die Fermentation in nährstofffreier Fumarsäurelösung wirkt sich wirtschaftlich günstig aus, vor allem dann, wenn die L-Äpfelsäure überwiegend oder ausschliesslich durch Fermentation in nährstofffreier Fumarsäurelösung hergestellt wird. Das Flüssigkeitsvolumen kann dabei das Einfache bis Zehnfache, vorzugsweise das Zweifache bis Fünffache des Volumens bei der Fermentation in nährstoffhaltiger Fumarsäurelösung oder bei der Züchtung in fumarsäurefreier oder fumarsäurehaltiger Nährstofflösung betragen. Das ermöglicht hinsichtlich der Volumina der beiden Arten
der Bio-Reaktoren ein Verhältnis von 1:1 bis 1:10.

Mit der aus einer Fermentation in nährstoffhaltiger Fumarsäurelösung oder aus einer Züchtung in
fumarsäurefreier oder fumarsäurehaltiger Nährstofflösung resultierenden Zellmasse können
mehrere, etwas bis zu drei (vorzugsweise zwei)
Fermentationen in nährstofffreier Fumarsäurelösung hintereinander durchgeführt werden. Wird
die Fermentation in nährstofffreier Fumarsäurelösung mehrfach wiederholt, empfiehlt es sich zur
Inaktivierung von dann eventuell auftretenden
Kontaminationskeimen, die Pilz-Zellmasse vor jedem erneuten Einsatz für 10 bis 60 Minuten in
einer Lösung mit einem tiefen pH-Wert, beispielsweise einem pH-Wert von 2 bis 3, eventuell unter
Zusatz eines Bakterizids, zu inkubieren. Derartige
Verfahren zur Bekämpfung von Kontaminationskeimen sind bereits von anderen biotechnologischen Verfahren (z. B. der Backhefe-Herstellung)
bekannt.

Die Erfindung hat folgende Vorteile:
– Es werden frei bewegliche Pilze und keine
Bakterien (in freier oder immobiliserter Form) sowie kein erst aus Zellmasse zu isolierendes Enzym
verwendet.
– Der Fermentationsansatz enthält Kalzium lediglich als Spurenelement (z. B. aus dem Trinkwasser).
– Die Fermentationszeit ist kurz, die Produktivität ist gross.
– Die Fumarsäure wird überwiegend oder ausschliesslich in nährstofffreier Lösung unter optimalen Fermentationsbedingungen umgesetzt; die
optimalen Wachstumsbedingungen des Pilzes
brauchen dabei nicht beachtet zu werden.
– Die abgetrennte Pilz-Zellmasse kann mehrfach
verwendet werden.
– Während der Fermentation in nährstofffreier
Fumarsäurelösung können sich Kontaminationskeime nicht nennenswert entwickeln.
– Die L-Äpfelsäure fällt nach Fermentation in
(nährstoffhaltiger oder nährstofffreier) Fumarsäurelösung in einer Konzentration von 100 bis 170 g
pro Liter an. Die Ausbeute beträgt mehr als 0,9 g L-
Äpfelsäure pro Gramm Fumarsäure, also mehr als
77% der theoretischen Ausbeute.
– L-Äpfelsäure wird in einer Lösung erhalten,
die deren Aufarbeitung zu einer für Nahrungsmittel und Pharmazeutika geeigneten Qualität sehr
nahe kommt, z. B. hinsichtlich der Abwesenheit
von LPS-Toxinen und Bernsteinsäure.
– Es entsteht keine D-Äpfelsäure.
– Das Verfahren ist sehr wirtschaftlich, da überwiegend Bio-Reaktoren einfacher Bauart verwendet werden.

Die Erfindung wird anhand der folgenden Beispiele erläutert, ohne auf diese beschränkt zu sein.

Die Fermentation in nährstoffhaltiger Fumarsäurelösung verläuft beispielsweise wie folgt:

**Beispiel 1**
In 1 Liter Trinkwasser werden 125 g Fumarsäure
vorgelegt und mit einer Mischung aus NaOH und
KOH (9 Teile NaOH und 1 Teil KOH) neutralisiert.
Folgende Nährstoffe werden zugegeben:
10 g Saccharose
1 ml konzentrierte Phosphorsäure ($H_3PO_4$)
1,4 ml konzentrierte Ammoniumhydroxidlösung
($NH_4OH$)
0,9 g Magnesiumsulfat ($MgSO_4 \cdot 7\,H_2O$)
30 mg Eisenchlorid ($FeCl_3 \cdot 6\,H_2O$)
Ein Fermenter mit 8 Liter Arbeitsvolumen wird
mit dieser nährstoffhaltigen Fumarsäurelösung
beschickt, thermisch sterilisiert und mit 0,25 Liter
Inoculum des Pilzes Aspergillus wentii geimpft.
Die Fermentationsbedingungen sind:
Belüfung: 0,5 Liter Luft pro Liter Reaktorvolumen und Minute (0,5 vvm).
Rührerdrehzahl: 500 Umdrehungen pro Minute
(Blattrührer in Kombination mit einem Leitrohr).
Temperatur: 30 °C.
pH-Wert: 7,0 (wird durch Einspeisung von
$NH_4OH$-Lösung mittels Steuerung über pH-Elektrode konstant gehalten).
Fermentationszeit: 72 Stunden.
Am Ende der Fermentationszeit sind 132 g L-
Äpfelsäure pro Liter Kulturlösung entstanden.

**Beispiel 2**
Die Fermentation nach Beispiel 1 wird unter
Erhöhung der Inoculum-Menge auf 0,8 Liter wiederholt. Nach 48 Stunden Fermentation sind 118 g
L-Äpfelsäure pro Liter Kulturlösung entstanden.

**Beispiel 3**
Die Fermentation nach Beispiel 1 wird unter
Einsatz von 0,8 Liter Inoculum des Pilzes Helminthosporium sativum wiederholt. Nach 72 Stunden
Fermentation sind 108 g L-Äpfelsäure pro Liter
Kulturlösung entstanden.

**Beispiel 4**
Die Fermentation nach Beispiel 1 wird unter
Einsatz von 0,8 Liter Inoculum des Pilzes Penicillium nalgiovensis wiederholt. Nach 72 Stunden
Fermentation sind 105 g L-Äpfelsäure pro Liter
Kulturlösung entstanden.

**Beispiel 5**
Die Fermentation nach Beispiel 1 wird mit einer
Nährlösung, die anstelle von Saccharose 10 g
Maismehl pro Liter Lösung enthält, wiederholt.
Nach 72 Stunden Fermentation sind 123 g L-Äpfelsäure pro Liter Kulturlösung entstanden.

**Beispiel 6**
Die Fermentation nach Beispiel 1 wird nach Erhöhung der Saccharosemenge auf 30 g pro Liter
wiederholt. Nach 72 Stunden Fermentation sind
128 g L-Äpfelsäure pro Liter Kulturlösung entstanden.
Zur Isolierung der während der Fermentation
gebildeten L-Äpfelsäure wird das Pilzmyzel durch
Filtrieren vom Kulturfiltrat getrennt und das Kulturfiltrat mit der stöchiometrischen Menge Salzsäure (bezogen auf die eingesetzte Fumarsäure) versetzt. Aus der salzsauren Lösung fällt die geringe Menge nicht umgesetzter Fumarsäure aus und wird abfiltriert. Das Filtrat wird nach Erwärmung auf ca. 60 °C mit der stöchiometrischen Menge Kalziumhydroxid (bezogen auf die gebildete L-Äpfelsäure) versetzt und 60 Minuten bei ca. 90 °C gerührt. Anschliessend wird heiss abfiltriert und das Ca-Malat schonend getrocknet. Man erhält aus 8 Liter Kulturfiltrat 1237 g Ca-Malat.

Die Fermentation in nährstofffreier Fumarsäurelösung verläuft beispielsweise folgendermassen:

Beispiel 7

Das durch Filtrieren der 8-Liter-Kulturfiltrat des Beispiels 6 erhaltene Pilzmyzel wird in 16 Liter einer 12,5%igen Fumarsäurelösung (neutralisiert mit NaOH und KOH im Verhältnis 9:1) suspendiert und in einem schwach gerührten und belüfteten Behälter bei 35 °C inkubiert. Nach 20 Stunden Fermentation sind 111 g L-Äpfelsäure pro Liter Kulturlösung entstanden.

Beispiel 8

Die Fermentation nach Beispiel 7 wird unter Zusatz von 1 g eines oberflächenaktiven Mittels, z. B. eines Emulgators, und 1 g Ammoniumsulfat $(NH_4)_2SO_4$ pro Liter Lösung wiederholt. Nach 16 Stunden Fermentation sind 115 g L-Äpfelsäure pro Liter Kulturlösung entstanden.

Beispiel 9

Die Fermentation nach Beispiel 7 wird mit 16 Liter einer 14,0%igen (mit NaOh und KOH – im Verhältnis 9:1 – neutralisierten) Fumarsäurelösung wiederholt. Nach 20 Stunden Fermentation sind 142 g L-Äpfelsäure pro Liter Kulturlösung entstanden.

Beispiel 10

Das aus der Fermentation nach Beispiel 7 abgetrennte Pilzmyzel wird in 16 Liter einer 12,5%igen Fumarsäurelösung (neutralisiert mit NaOH und KOH im Verhältnis 9:1) suspendiert. Nach 20 Stunden Fermentation sind 113 g L-Äpfelsäure pro Liter Kulturlösung entstanden.

Beispiel 11

Das aus der Fermentation nach Beispiel 10 abgetrennte Pilzmyzel wird für eine weitere Fermentation analog zu Beispiel 10 eingesetzt. Nach 24 Stunden Fermentation sind 108 g L-Äpfelsäure pro Liter Kulturlösung entstanden.

Bei allen Beispielen wird die Kulturlösung analog zu Beispiel 6 aufgearbeitet.

## Patentansprüche

1. Verfahren zur Herstellung des reinen L-Isomeren der Äpfelsäure aus neutralisierter Fumarsäure durch mikrobielle Fermentation mittels frei beweglicher Mikroorganismen in wässriger Phase, wobei eine Konzentration der L-Äpfelsäure zum Zeitpunkt der Ernte von 100 bis 170 g pro Liter der Kulturlösung, gekennzeichnet durch

– Verwenden von Pilzen als Mikroorganismen,
– einer Konzentration der vorgelegten Fumarsäure von 11 bis 15 Gew.-%,
entsteht.

2. Verfahren nach Anspruch 1, gekennzeichnet durch
– Herstellen einer wässrigen Lösung der neutralisierten Fumarsäure, die zusätzlich die für das Wachsen des Pilzes nötigen Nährstoffe enthält,
– Impfen der Lösung mit 1 bis 20% Inoculum des Pilzes,
– Fermentieren bei 20 bis 50 °C während 1 bis 3 Tagen,
– Abtrennen der Zellmasse und der L-Äpfelsäure aus der Kulturlösung.

3. Verfahren nach Anspruch 1, gekennzeichnet durch
– Herstellen einer wässrigen Lösung der neutralisierten Fumarsäure ohne Nährstoffe für den Pilz,
– Zugeben der aus einer Züchtung in fumarsäurefreier oder fumarsäurehaltiger Nährlösung oder aus einer Fermentation in nährstoffhaltiger Fumarsäurelösung resultierenden Zellmasse zu der nährstofffreien Fumarsäurelösung, deren Volumen das Einfache bis Zehnfache des Flüssigkeitsvolumens der vorangegangenen Züchtung in fumarsäurefreier oder fumarsäurehaltiger Nährlösung oder der vorangegangenen Fermentation in nährstoffhaltiger Fumarsäurelösung ausmacht, wenn die gesamte Zellmasse der vorausgegangenen Züchtung oder Fermentation eingesetzt wird,
– Fermentieren während 12 bis 24 Stunden bei 25 bis 60 °C,
– Abtrennen der Zellmasse und der L-Äpfelsäure aus der Kulturlösung.

4. Verfahren nach den Ansprüchen 1 bis 3, gekennzeichnet durch
– mehrfaches Verwenden der abgetrennten Zellmasse in einer jeweils neu angesetzten nährstofffreien Fumarsäurelösung.

5. Verfahren nach den Ansprüchen 1 bis 4, gekennzeichnet durch
– Verwenden von Pilzen aus einer der Ordnungen Apsergillus, Penicillium, Paecilomyces, Trichosporon, Taphrina, Helminthosporium, Pythium.

6. Verfahren nach den Ansprüchen 1 bis 5, gekennzeichnet durch
– Verwenden des Pilzes Aspergillus wentii.

## Claims

1. A process for producing the pure L-isomer of malic acid from neutralized fumaric acid under bioconversion conditions by free movable microorganisms in an aqueous solution of fumarate characterized in that said microorganisms are fungi, that the initial concentration of fumarate is 11 to 15% by weight and that the concentration of L-malic acid at the time of harvesting is 100 to 170 g per liter of fermentation broth.

2. A process according to claim 1 wherein an aqueous solution of neutralized fumaric acid is prepared which further contains nutrients for said fungus and which is inoculated with a solution of 1 to 20% of an inoculum of said fungus and wherein the incubation is maintained for 1–3 days at 20–50 °C and the cell mass and the resultant L-malic acid are recovered.

3. A process according to claim 1 wherein an aqueous solution of neutralized fumaric acid is prepared, without any nutrients for the fungus, and wherein a cell mass is added to the nutrient-free solution of fumaric acid, said cell mass being recovered from the fermentation of a nutrient-containing solution of fumaric acid or from the proliferation within a nutrient solution free of fumaric acid or containing fumaric acid, the volume of said nutrient-free solution of fumaric acid being about 1 to 10 times the volume of the liquid used during the previous proliferation in a nutrient solution with or without fumaric acid or used during a previous fermentation in a nutrient-containing solution of fumaric acid if the whole cell mass of the previous proliferation or fermentation is used, and wherein incubation is maintained for 12–24 hours at 25–60 °C and the cell mass and the resultant L-malic acid are recovered.

4. A process according to claims 1 to 3 wherein the recovered cell mass il utilized repeatedly for fermentation of a freshly prepared nutrient-free solution of fumaric acid.

5. A process according to claims 1 to 4 wherein said fungus belongs to the genus Aspergillus, Penicillium, Paecilomyces, Trichosporon, Taphrina, Helminthosporium, or Pythium.

6. A process according to claims 1 to 5, wherein said fungus belongs to the species Aspergillus wentii.

## Revendications

1. Procédé de préparation à l'état pur de l'isomère lévogyre de l'acide malique à partir d'acide fumarique neutralisé, par fermentation microbiologique à l'aide de micro-organismes librement mobiles en phase aqueuse, ledit procédé étant caractérisé par:
– l'utilisation de champignons en tant que micro-organismes,
– une concentration de 11 à 15% en poids de l'acide fumarique utilisé,
tandis que s'établit, au moment de la récolte, une concentration de l'acide malique lévogyre de 100 à 170 g par litre de la solution de culture.

2. Procédé selon la revendication 1, caractérisé par:
– la préparation d'une solution aqueuse de l'acide fumarique neutralisé, qui renferme complémentairement les substances nutritives nécessaires pour la croissance du champignon,
– l'ensemencement de la solution avec 1 à 20% d'inoculum du champignon,
– la fermentation pendant un à trois jours à une température de 20 à 50 °C,
– la séparation de la masse cellulaire et de l'acide malique lévogyre à partir de la solution de culture.

3. Procédé selon la revendication 1, caractérisé par:
– la préparation d'une solution aqueuse de l'acide fumarique neutralisé, sans substances nutritives pour le champignon,
– l'addition, à la solution d'acide fumarique exempte de substance nutritive, de la masse cellulaire résultant d'une culture dans la solution nutritive exempte d'acide fumarique ou en renfermant, ou provenant d'une fermentation dans une solution d'acide fumarique renfermant de la substance nutritive, le volume de la solution d'acide fumarique exempte de substance nutritive étant d'une à dix fois le volume de liquide de la culture préalable dans la solution nutritive exempte d'acide fumarique ou en renfermant ou du volume de la fermentation préalable dans la solution d'acide fumarique renfermant de la substance nutritive, lorsque la masse cellulaire globale, provenant de la culture ou de la fermentation préalable, est utilisée,
– la fermentation pendant 12 à 24 heures à une température de 25 à 60 °C,
– la séparation, à partir de la solution de culture, de la masse cellulaire et de l'acide malique lévogyre.

4. Procédé selon les revendications 1 à 3, caractérisé par:
– l'utilisation répétée de la masse cellulaire séparée dans une solution d'acide fumarique exempte de solution nutritive qui est à nouveau chaque fois utilisée.

5. Procédé selon les revendications 1 à 4, caractérisé par:
– l'utilisation de champignons de l'un des groupes Aspergillus, Penicillium, Paecilomyces, Trichosporone, Taphrina, Helminthosporium, Pythium.

6. Procédé selon les revendications 1 à 5, caractérisé par:
– l'utilisation du champignon Aspergillus ventii.